Europäisches Patentamt

European Patent Office

Office européen des brevets

(19)

(11) Veröffentlichungsnummer: **0 131 001**
**B1**

(12) # EUROPÄISCHE PATENTSCHRIFT

(45) Veröffentlichungstag der Patentschrift:
**20.11.86**

(51) Int. Cl.⁴: **C 07 C 47/542,** C 07 C 45/28,
**C 25 B 1/00**

(21) Anmeldenummer: **84900023.7**

(22) Anmeldetag: **19.12.83**

(86) Internationale Anmeldenummer:
**PCT/CH 83/00145**

(87) Internationale Veröffentlichungsnummer:
**WO 84/02522 (05.07.84** Gazette **84/16)**

(54) **VERFAHREN ZUR HERSTELLUNG EINES ALDEHYDS.**

(30) Priorität: **29.12.82 CH 7612/82**
**03.11.83 CH 5934/83**

(43) Veröffentlichungstag der Anmeldung:
**16.01.85 Patentblatt 85/3**

(45) Bekanntmachung des Hinweises auf die Patenterteilung:
**20.11.86 Patentblatt 86/47**

(84) Benannte Vertragsstaaten:
**AT BE CH DE FR GB LI NL**

(56) Entgegenhaltungen:
**DE - A - 2 435 985**
**FR - A - 338 990**
**FR - A - 2 462 411**

**Journal of the Electrochemical Society, (1963) Vol. 110,
No. 3, R. Ramaswamy et al. "Electrolytically
Regenerated Manganic Sulfate for the Oxydation of
Aromatic Hydrocarbons", pages 202-204, see page 203
cited in the application**
**Journal of the Electrochemical Society, (1982) Vol. 129,
No. 4 Ch. Comminellis et al.: "Electrochemical**

(73) Patentinhaber: **L. GIVAUDAN & CIE Société Anonyme,
CH-1214 Vernier-Genève (CH)**

(72) Erfinder: **COMNINELLIS, Christos, 38 Floréal,
CH-1008 Prilly (CH)**
Erfinder: **PLATTNER, Eric, 18 Jurastrasse,
CH-4411 Seltisberg (CH)**

(74) Vertreter: **Lederer, Franz, Dr. et al, Vanderwerth, Lederer
& Riederer Patentanwälte Lucile-Grahn-Strasse 22,
D-8000 München 80 (DE)**
Vertreter: **Urech, Peter, Dr. et al,
Grenzacherstrasse 124 Postfach 3255, CH-4002 Basel
(CH)**

(56) Entgegenhaltungen: (Fortsetzung)
**Production of Manganic Sulfate in Concentrated H2S04,
page 749-752, see page 749 cited in the application**

**Die Akte enthält technische Angaben, die nach dem
Eingang der Anmeldung eingereicht wurden und die
nicht in dieser Patentschrift enthalten sind.**

## Beschreibung

Die Erfindung betrifft ein Verfahren zur Herstellung von p-tert. Butylbenzaldehyd (TBB). Dieser Aldehyd ist eine bekannte Substanz, insbesondere mit Zwischenproduktcharakter.

Das Verfahren ist dadurch gekennzeichnet, dass man p-tert-Butyltoluol (TBT) mit einem $Mn^{3+}$-Salz bei Temperaturen von 90°C bis 110°C oxidiert, welches Salz durch elektrochemische Oxidation eines $Mn^{2+}$-Salzes bei Temperaturen von 60°C bis 110°C und bei Stromdichten von $\geq 100$ mA/cm$^2$ erzeugt wird, und wobei chemische Oxidation und elektrochemische Oxidation in separaten Reaktionsgefässen durchgeführt werden.

Als Mangansalz wird vorzugsweise das Sulfat verwendet. Aber auch das Phosphat kann verwendet werden. Auch dieses Salz bietet bzgl. elektrochemischer Stabilität — und zwar kathodisch wie anodisch — bzgl. Interferenz mit organischem Material und bzgl. Korrosion keinerlei Probleme.

Die elektrochemische Oxidation wird zweckmässigerweise in mässig konzentrierter, nämlich in 40 (5,3 molarer) bis 90%iger (16,6 molarer), insbesondere in 50 (7,1 molarer) bis 65%iger (10,3 molarer) Schwefelsäure durchgeführt.

Man arbeitet, wie angegeben bei erhöhten Temperaturen, vorzugsweise in einem Temperaturbereich von 80 bis 100°C, besonders bei etwa 85°C.

Die Konzentration des Mangansalzes in der anorganischen Phase beträgt zweckmässigerweise 1 bis 5 Mol/l, insbesondere 3,0 bis 4,9 Mol/l, besonders etwa 3 Mol/l.

Als Anodenmaterial kann im Prinzip jedes Elektrodenmaterial eingesetzt werden, das unter den Verfahrensbedingungen beständig ist, also z.B.:

glasartiger Graphit, Blei, Bleilegierungen oder Edelmetalle wie Platin, oder edelmetallbeschichtete, z.B. mit Palladium oder Ruthenium, gegen anodische Korrosion passive Metalle, wie Zirkon und Tantal.

Besonders bevorzugt sind Bleilegierungen, z.B. solche mit einem Gehalt an Silber , z.B. Chromin von Blasberg, BRD. Im Elektrolyten finden sich auf diese Weise als Katalysator wirkende Ag$^-$-Ionen, also die niedriger wertigen Metallionen eines Übergangsmetall-Redox-Paares mit einem Oxidationspotential $> Mn^{2+}/Mn^{3+}$. Es hat sich gezeigt, dass insbesondere das Arbeiten mit solchen Bleilegierungen wirtschaftlich ist, weil die Standzeit (Lebensdauer) der Elektroden hoch ist — z.B. 1 bis 1$^{1}/_{2}$ Jahre betragen kann.

Als Kathodenmaterial wird ebenfalls glasartiger Graphit, Blei, Bleilegierungen, Edelmetalle wie Platin, insbesondere aber ebenfalls Blei oder Bleilegierungen, z.B. Chromin verwendet.

Die Reaktion kann in einer ungeteilten oder in einer durch eine aus üblichen inerten Materialien bestehende, poröse Zwischenwand (Diaphragma) geteilten Zelle durchgeführt werden. Vorzugsweise arbeitet man in einer Zelle ohne Diaphragma. Obschon es in letzterem Falle zweckmässig ist, zum Schutz vor Knallgasbildung (aus anodisch gebildeten $O_2$ und kathodisch erzeugtem $H_2$) in einer Schutzgasatmosphäre, z.B. unter $N_2$ zu arbeiten, kann man auch einfach die bei der Reaktion entstehenden Gase während der Elektrolyse durch Zugabe eines Schutzgases, z.B. $N_2$ verdünnen; aber auch Verdünnen mit Luft führt schon zur Verhinderung der Explosionsgefahr.

Für die elektrochemische Oxidation kann eigentlich jeder gängige — insbesondere marktgängige — Zellentyp verwendet werden, man kann also z.B. mit

— Kanalzellen (in welcher die Elektroden als Kamm oder als Paket [in Form von Platten, Zylindern, etc.] angeordnet sind) [bevorzugt]

— Filterpressenzellen (bestehend aus Rahmen und Platten)

— Trogzellen (wobei die nötige Rührwirkung durch Elektrolytzirkulation oder mittels Inertgas oder mittels Drehelektroden realisiert wird, etc.)

arbeiten. Ein bevorzugter Kanalzellentyp ist z.B. von D. Pletcher, in Industrial Electrochemistry, Chapman & Hall (London, New York), (1982), 162, beschrieben worden.

Als Zellenmaterial kann jedes inerte Material, insbesondere aber Kunststoffe auf Polymerbasis, z.B. Polypropylen verwendet werden.

Die Stromdichten in der erfindungsgemässen Reaktion liegen zweckmässigerweise bei 100 bis 600 mA/cm$^2$, insbesondere bei 300 bis 500 mA/cm$^2$, d.h. 3 bis 5 kA/m$^2$.

Die Zellenspannung stellt sich in Abhängigkeit von der Zusammensetzung des Elektrolyten, der Temperatur und der Geometrie der Einzelzelle ein. Sie kann beispielsweise Werte von 2,5 bis 4 V, insbesondere von 2,5 bis 3,0 V, besonders etwa 3 V annehmen.

Die Oxidation von p-tert. Butyltoluol wird erfindungsgemäss mit einem elektrochemisch erzeugten $Mn^{3+}$-Salz — aber in einem separaten Reaktionsgefäss — durchgeführt.

Vorzugsweise arbeitet man dabei in einem Dreiphasensystem, nämlich: Schwefelsäure/darin suspendiertem $Mn^{3+}$-(u. $Mn^{2+}$)Salz/organisches Material.

Die Schwefelsäurekonzentration beträgt vorzugsweise 40 bis 90%, insbesondere 50 bis 65%, besonders etwa 55%.

Der Gehalt an Mangansalzen ($Mn^{+2}$[Spuren] und $Mn^{+3}$) in der anorganischen Phase liegt zweckmässigerweise bei mindestens 2,5 Mol/l, beträgt also z.B. 3 bis 4 Mol/l, insbesondere etwa 3 Mol/l.

Das organische Material besteht hauptsächlich aus TBT, TBB und Lösungsmittel. Als Lösungsmittel kommen insbesondere aliphatische Kohlenwasserstoffe — z.B. Heptane, Oktane, Cyclohexan — sowie ihre chlorierten oder fluorierten Derivate in Frage, z.B. Methylenchlorid, Tetrachloräthylen, Perfluorooktan ($C_8F_8$), etc. Diese Lösungsmittel weisen nämlich ein ausreichendes Lösevermögen für TBT und seine Oxidationsprodukte auf; sie sind unlöslich in den anorganischen Phasen; ihre chemische Stabilität gegenüber dem Reaktionsmilieu ist befriedigend, und sie sind leicht von den Reaktionsprodukten abtrennbar. Bevorzugt werden Oktane und chlorierte Aliphaten. Besonders bevorzugt ist TBT.

Die Reaktionstemperatur beträgt 80°C bis 110°C, insbesondere 90 bis 100°C, besonders etwa 95°C.

Es ist vorteilhaft, die chemische Oxidation nach einem Umsatz von $\leq 40\%$, insbesondere nach 20 bis 30% p-tert. Butyltoluol abzubrechen, insbesondere wegen der Gefahr der Abnahme der Selektivität. Diese beträgt im optimalen Fall über 90%. Als Nebenprodukte sind insbesondere geringe Mengen, z.B. ~3% der entsprechenden Carbonsäure, möglich.

Die Trennung des im wesentlichen aus TBB, $MnSO_4$, Schwefelsäure und organischem Lösungsmittel bestehenden Reaktionsproduktes in zwei anorganische Phasen (Suspension von $MnSO_4 \cdot H_2O$ in Schwefelsäure) und eine organische Phase kann durch Filtrieren und Dekantieren oder durch Zentrifugieren bewerkstelligt werden. Die Trennung von TBB und restlichem organischem Material bzw. Lösungsmittel erfolgt zweckmässigerweise destillativ.

Das TBT kann hierauf wieder in den Kreislauf zurückverbracht werden.

Vor Rezyklieren der anorganischen Phase wird diese zweckmässigerweise mittels Wasserdampf und/oder einem Gas von restlichem organischem Material befreit. Am einfachsten geschieht dies durch Stripping in Kolonnen im Gegenstromverfahren.

Im Falle der elektrochemischen Oxidation arbeitet man vorzugsweise kontinuierlich, im Falle der chemischen Oxidation kann kontinuierlich oder diskontinuierlich gearbeitet werden.

Die Stromausbeute des elektrochemischen Prozesses liegt über 70%.

Die elektrochemische Oxidation von $Mn^{2+}$ zu $Mn^{3+}$ als solche ist zwar aus der Literatur bekannt, siehe z.B. J. Electrochem. Soc. *129* [4], 749-752 (1982).

Es ist auch bekannt, p-tert. Butyltoluol direkt elektrolytisch zu p-tert. Butylbenzaldehyd zu oxidieren, siehe z.B. die japanischen Patentanmeldungen Nr. 79/096 296 und 79/56 996 und die DOS 2 948 455.

Es ist weiter bekannt, dass aromatische Kohlenwasserstoffe, z.B. Xylole oder Toluole unter Verwendung von elektrolytisch hergestelltem $Mn_2(SO_4)_3$ oxidiert werden können, siehe z.B. die deutsche Patentschrift 175 295, die US-Patentschriften 4 212 710 und 4 212 711 und R. Ramaswamy et al., J. Electrochemical Society *110* [3], 202-204, (1963).

Keiner dieser Literaturstellen können die oben aufgeführten Parameter, die es gestatten, den erfindungsgemäss erhältlichen Aldehyd selektiv in grosstechnischem Massstab wirtschaftlich, also preisgünstig herzustellen, entnommen werden. Diesbezüglich ist das vorliegende Verfahren auch dem Verfahren unter Verwendung von $Ce^{4+}$-Salzen eindeutig überlegen (siehe z.B. die deutsche Patentschrift 3 028 757). Verglichen mit diesem Verfahren gestatten insbesondere folgende Parameter ein wirtschaftlicheres Verfahren: niedrigeres Atomgewicht, Preis, Stromausbeute, Konzentration des Salzes bei der elektrochemischen Oxidation ~ Konzentration bei der chemischen Oxidation, Stabilität des Lösungsmittels im Anodenraum, niedrigere Zellenspannung, höhere Konzentration des Salzes bei der elektrochemischen Oxidation im erfindungsgemässen Fall, Aufarbeitung des (chemisch) hergestellten tert. Butylbenzaldehydes. Ein besonderer Vorteil des Arbeitens mit Mangansalzen ist auch darin zu erblicken, dass im Rahmen der vorliegenden Erfindung gefunden

wurde, dass $Mn^{3+}$ in etwa 59%iger Schwefelsäure rund 10 mal weniger löslich ist als $Mn^{2+}$; mit anderen Worten, die Gefahr der Reduktion von $Mn^{3+}$ an der Kathode ist während der elektrochemischen Oxidation recht gering.

Obschon also der einschlägige Stand der Technik eine beträchtliche Anzahl Beiträge zum vorliegenden Problem aufweist, fehlte bisher ein diesbezügliches Verfahren, das erlaubt hätte, den Aldehyd TBB so selektiv und effizient herzustellen, um als Betriebsverfahren in Frage zu kommen.

Es kann diesbezüglich auch noch auf DT-OS 2 435 985 (Hauck et al., Oxy Metal Industries Corp.) hingewiesen werden.

Hauck et al. beschreiben u.a. die Herstellung von Anisaldehyd bzw. p-Isopropylbenzaldehyd durch Oxydation von p-Methoxy- bzw. p-Isopropyltoluol; es wird hier darauf hingewiesen, dass zweck Herstellung der Aldehyde die Temperatur bei der chemischen Stufe nicht wesentlich über 30°C liegen sollte, und dass die Schwefelsäurekonzentration 2,5 bis 8,5 Gew.-% entsprechen sollte. Höhere Temperaturen und höhere Säurekonzentrationen liefern Gemische von Säuren und Aldehyden (Seiten 10-12). Die diesbezügliche Lehre deckt sich im wesentlichen mit der Lehre von Ramaswamy et al., siehe oben.

*Beispiel 1*

| Apparatur: | ungeteilte Zirkulations-Plattenzelle und Behälter von 1,5 l Inhalt (Kanalzelle) |
|---|---|
| Anode und Kathode: | Chromin (Blasberg), Oberfläche 182 cm² |
| Elektrolyt: | 454 g $MnSO_4 \cdot H_2O$ 428 g $H_2O$ 699 g $H_2SO_4$ 100% |
| Temperatur: | 95-100°C |
| Stromstärke: | 39 A |
| Spannung: | 2,5-3,0 V |

Nach Elektrolyse mit einer Strommenge von 3,58 F (96 A · h) erhält man 725 g $Mn_2(SO_4)_3 \cdot H_2SO_4 \cdot 4H_2O$, die Stromausbeute beträgt 71,5%, Umsatz bzgl. $Mn^{2+}$ = 95%.

Der Elektrolyt wird nun in ein Reaktionsgefäss von 1,5 l gegeben. Dazu gibt man unter Rühren 135 ml Wasser innert 10 Minuten zu und erhitzt auf 90°C. Man gibt nun auf einmal 360 g auf 60° erhitztes TBT zu, wobei die Temperatur zunächst auf 80° absinkt und hierauf auf 95° ansteigt. Das Volumen des Reaktionsgemisches beträgt 1,4 l. Man rührt 15 Minuten bei 95°C weiter und saugt die flüssigen Phasen durch eine Glasfritte in einen auf 95°C thermostatisierten Scheidetrichter. Nach Trennung der flüssigen Phasen wird die anorganische Phase in das Reaktionsgefäss zurückverbracht, man rührt kurz und saugt wieder ab. Nach dreimaligem Absaugen erhält man 345 g organische Phase und 1,71 kg anorganische Phase, letztere mit geringem Gehalt an organischer Beimengung.

Die organische Phase wird mit 180 g einer 5%igen $Na_2CO_3$-Lösung (enthaltend 5% NaCl) und mit 90 g einer 10%igen NaCl-Lösung gewaschen. Die organische Phase wird an einer Vigreux-Kolonne unter Vakuum destilliert und ergibt 270 g TBT als niedriger siedende Komponente und 83,6 g TBB als höher siedende Komponente.

Ausbeute:

TBT-Verbrauch: 360-270 = 90 g
TBB destilliert: 83,6 g = entsprechend 85% d.TH.
Overall-Stromausbeute: 71,5 · 80,66 = 57,8%.

Die anorganische Phase kann nach Abdestillieren von ca. 125 g Wasser und wenig organischem Material an Stelle von frischem Elektrolyten in den Elektrolysekreislauf zurückgeführt werden.

### Beispiel 2

Apparatur:    ungeteilte Zirkulations-Plattenzelle und Behälter von 1,5 l Inhalt

Anode und
Kathode:     Chromin (Blasberg), Oberfläche 182 cm²

Elektrolyt:    454 g $MnSO_4 \cdot H_2O$
              428 g $H_2O$
              699 g $H_2SO_4$ 100%

Temperatur:  95-100°C
Stromstärke: 39 A
Spannung:    2,5-3,0 V

Nach Elektrolyse mit einer Strommenge von 3,58 F (96 A · h) erhält man 725 g $Mn_2(SO_4)_3 \cdot H_2SO_3 \cdot 4H_2O$, die Stromausbeute beträgt 71,5%, Umsatz bzgl. $Mn^{2+}$ = 95%.

In einem 1,5 l Reaktionsgefäss werden 360 g TBT auf 90°C erhitzt. Man gibt nun innerhalb 1 bis 1$^1/_2$ Stunden den oxidierten Elektrolyten unter gutem Rühren zu. Gleichzeitig werden dem Reaktionsgefäss 135 ml Wasser zugeführt. Die Temperatur wird während der Zugabe und der Nachrektionszeit (40 Minuten) bei 95°C gehalten. Die Reaktionsmasse wird auf 60°C gekühlt, darauf werden die flüssigen Phasen durch eine Glasfritte in einen auf 60°C thermostatisierten Scheidetrichter abgesogen. Nach Trennung der flüssigen Phasen wird die anorganische Phase in das Reaktionsgefäss zurückverbraucht, man rührt kurz und saugt wieder ab. Nach dreimaligem Absaugen erhält man 345 g organische Phase und 1,71 kg anorganische Phase, letztere mit geringem Gehalt an organischer Beimengung.

Die organische Phase wird bei 60°C mit ca. 180 g einer 20%igen, neutralen $Na_2SO_4$-Lösung gewaschen. Diese Lösung kann mehrmals benützt werden, indem ihr pH-Wert mit wenig 12%iger Natronlauge immer auf 7 ± 0,5 gebracht wird. Die organische Phase wird an einer Vigreux-Kolonne unter Vakuum destilliert und ergibt 270 g TBT als niedriger siedende Komponente und 86,0 g TBB als höher siedende Komponente.

Ausbeute:

TBT-Verbrauch: 360 - 270 = 90 g
TBB destilliert: 86,0 g = entsprechend 87% d.TH.
Overall-Stromausbeute: 71,5 · 83,2 = 59,5%.

Die anorganische Phase kann nach Abdestillieren von ca. 125 g Wasser und wenig organischem Material an Stelle von frischem Elektrolyten in den Elektrolysekreislauf zurückgeführt werden.

### Beispiel 3

Im Betrieb kann in einem ersten Reaktionssystem kontinuierlich die elektrochemische Oxidation $Mn^{2+}$ → $Mn^{3+}$ (in $H_2SO_4$) durchgeführt werden und das erhaltene Gemisch $Mn^{3+}/H_2SO_4$ in ein zweites Reaktionsgefäss übergeführt werden, um dort die chemische Oxidation TBT → TBB unter Zugabe eines organischen Lösungsmittels in einem 3-Phasensystem (Schwefelsäure/darin suspendiertes $Mn^{3+}$/organisches Lösungsmittel), z.B. TBT) durchzuführen. Das erhaltene Reaktionsprodukt wird in zwei anorganische Phasen (Suspension von $MnSO_4 \cdot H_2O$ in Schwefelsäure) und eine organische Phase durch Zentrifugieren aufgetrennt. Die anorganische Phase wird durch Stripping (z.B. mit $H_2O$-Dampf) in einer Füllkörperkolonne im Gegenstromverfahren vom restlichen organischen Material befreit und schliesslich wieder in das erste Reaktionssystem zurückgeführt. Die organische Phase wird alkalisch gewaschen (wie oben beschrieben) und durch Destillation in rohes TBB und organisches Lösungsmittel aufgetrennt. Das durch Stripping gewonnene TBT, TBB und $H_2O$ wird in das System, z.B. dem zweiten Reaktionsgefäss, zugeführt.

### Patentansprüche

1. Verfahren zur Herstellung von p-tert. Butylbenzaldehyd, dadurch gekennzeichnet, dass man p-tert. Butyltoluol mit einem $Mn^{3+}$-Salz bei Temperaturen von 80°C bis 110°C, oxidiert, welches Salz durch elektrochemische Oxidation eines $Mn^{2+}$-Salzes bei Temperaturen von 60°C bis 110°C und bei Stromdichten von ≥ 100 mA/cm² erzeugt wird, und wobei chemische Oxidation und elektrochemische Oxidation in separaten Reaktionsgefässen durchgeführt werden.

2. Verfahren nach Anspruch 1, dadurch gekennzeichnet, dass unter Verwendung von Mangansulfat gearbeitet wird.

3. Verfahren nach Anspruch 1 oder 2, dadurch gekennzeichnet, dass man die elektrochemische Oxidation in 40 bis 90%iger, insbesondere in 50 bis 65%iger, Schwefelsäure durchführt.

4. Verfahren nach einem der Ansprüche 1-3, dadurch gekennzeichnet, dass man $Mn^{2+}$ bei einer Temperatur von 80 bis 100°C, elektrochemisch oxidiert.

5. Verfahren nach einem der Ansprüche 1-4, dadurch gekennzeichnet, dass das $Mn^{2+}$-Salz in einer Konzentration von 1 bis 5 Mol/l, insbesondere von 3,0 bis 4,0 Mol/l, in der anorganischen Phase vorliegt.

6. Verfahren nach einem der Ansprüche 1-5, dadurch gekennzeichnet, dass man bei hohen Stromdichten von 300 bis 500 mA/cm², arbeitet.

7. Verfahren nach einem der Ansprüche 1-6, dadurch gekennzeichnet, dass man bei einer Zellenspannung von etwa 2,5 bis 4 V, insbesondere 2,5 bis 3,0 V, arbeitet.

8. Verfahren nach einem der Ansprüche 1-7, dadurch gekennzeichnet, dass als Anodenmaterial glasartiger Graphit, Blei, Beilegierungen, Edelmetalle wie Platin oder edelmetallbeschichtete, gegen anodische Korrosion passive Metalle, insbesondere aber Bleilegierungen, verwendet werden.

9. Verfahren nach einem der Ansprüche 1-8, dadurch gekennzeichnet, dass man als Kathodenmaterial glasartigen Graphit, Blei, Bleilegierungen oder Edelmetalle, insbesondere aber Blei oder Bleilegierungen verwendet.

10. Verfahren nach Anspruch 8 oder 9, dadurch gekennzeichnet, dass Anode und Kathode aus demselben Material bestehen.

11. Verfahren nach Anspruch 10, dadurch gekennzeichnet, dass eine Bleisilberlegierung, insbesondere Chromin, als Elektrodenmaterial verwendet wird.

12. Verfahren nach einem der Ansprüche 1-11, dadurch gekennzeichnet, dass man die Reaktion in einer ungeteilten Elektrolysezelle, d.h. in einer Zelle ohne Diaphragma, durchgeführt.

13. Verfahren nach einem der Ansprüche 1-12, dadurch gekennzeichnet, dass man in Filterpressenzellen, Trogzellen oder Kanalzellen, insbesondere in Kanalzellen arbeitet.

14. Verfahren nach einem der Ansprüche 1-13, dadurch gekennzeichnet, dass die Zelle aus inertem Material, insbesondere aus Kunststoffen auf Polymerbasis besteht.

15. Verfahren nach einem der Ansprüche 1-14, dadurch gekennzeichnet, dass man die chemische Oxidation in einem Dreiphasensystem durchführt.

16. Verfahren nach Anspruch 15, dadurch gekennzeichnet, dass zwei der drei Phasen dem System $Mn^{3+}(Mn^{2+})/H_2SO_4$ der elektrochemischen Oxidation entsprechen.

17. Verfahren nach Anspruch 15 oder 16, dadurch gekennzeichnet, dass das Dreiphasensystem aus einer Suspension von festem $Mn^{2+}$-, bzw. $Mn^{2+}$- und $Mn^{3+}$-Salz in 40 bis 90%iger $H_2SO_4$, insbesondere in 50 bis 65%iger $H_2SO_4$, und organischem Material besteht.

18. Verfahren nach Anspruch 17, dadurch gekennzeichnet, dass die Gesamtmenge an Mangansalzen in der anorganischen Phase $\geq$ 2,5 Mol/l, insbesondere 3 bis 4 Mol/l, beträgt.

19. Verfahren nach einem der Ansprüche 15-18, dadurch gekennzeichnet, dass man p-tert.Butyltoluol als Lösungsmittel verwendet.

20. Verfahren nach Anspruch 19, dadurch gekennzeichnet, dass das p-tert.Butyltoluol bei einer Temperatur von 90 bis 100°C oxidiert wird.

21. Verfahren nach einem der Ansprüche 1-20, dadurch gekennzeichnet, dass man unter Schutzgasatmosphäre, z.B. unter $N_2$, arbeitet.

22. Verfahren nach einem der Ansprüche 1-21, dadurch gekennzeichnet, dass man die Oxidation nach einem Umsatz von $\leq$ 40%, insbesondere 20 bis 30% p-tert. Butyltoluol abbricht.

23. Verfahren nach einem der Ansprüche 1-22, dadurch gekennzeichnet, dass man die Trennung des im wesentlichen aus TBB, $MnSO_4$, $H_2SO_4$ und organischem Lösungsmittel bestehenden Reaktionsproduktes in die anorganischen Phasen und die organische Phase durch Filtrieren und Dekantieren oder durch Zentrifugieren bewerkstelligt.

24. Verfahren nach einem der Ansprüche 1-23, dadurch gekennzeichnet, dass die anorganischen Phasen vor der Rückführung in den Elektrolysekreislauf von organischem Material befreit werden, insbesondere durch Stripping mittels Dampf oder einem inerten Gas.

25. Verfahren nach einem der Ansprüche 1-24, dadurch gekennzeichnet, dass die gereinigten anorganischen Phasen an Stelle frischen Elektrolyts in den Elektrolysekreislauf zurückgeführt werden.

26. Verfahren nach einem der Ansprüche 1-25, dadurch gekennzeichnet, dass man bei der elektrochemischen Oxidation kontinuierlich und bei der chemischen Oxidation kontinuierlich oder diskontinuierlich arbeitet.

**Claims**

1. A process for the manufacture of p-tert.butylbenzaldeide, characterized by oxidizing p-tert.butyltoluene with a $Mn^{3+}$ salt at temperatures of 80°C to 110°C, which salt is produced by the electrochemical oxidation of a $Mn^{2+}$ salt at temperatures of 60°C to 110°C and at current densities of $\geq$ 100 mA/cm$^2$, and whereby the chemical oxidation and the electrochemical oxidation are carried out in separate reaction vessels.

2. A process according to claim 1, characterized in that it is carried out using manganese sulphate.

3. A process according to claim 1 or 2, characterized in that the electrochemical oxidation is carried out in 40 to 90%, especially in 50 to 65%, sulphuric acid.

4. A process according to any one of claims 1-3, characetrized in that $Mn^{2+}$ is oxidized electrochemically at a temperature of 80 to 100°C.

5. A process according to any one of claims 1-4, characterized in that the $Mn^{2+}$ salt is present in a concentration of 1 to 5 mol/l, especially of 3.0 to 4.0 mol/l in the inorganic phase.

6. A process according to any one of claims 1-5, characterized in that it is carried out with high current densities of 300 to 500 mA/cm$^2$.

7. A process according to any one of claims 1-6, characterized in that it is carried out at a cell potential of about 2.5 to 4 V, especially 2.5 to 3.0 V.

8. A process according to any one of claims 1-7, characterized in that vitreous graphite, lead, lead alloys, noble metals such as platinum of noble metal-coated metals which are passive towards anodic corrosion, especially lead alloys, is/are used as the anode material.

9. A process according to any one of claims 1-8, characterized in that vitreous graphite, lead, lead alloys or noble metals, especially lead or lead alloys, is/are used as the cathode material.

10. A process according to claim 8 or 9, characterized in that the anode and cathode consist of the same material.

11. A process according to claim 10, characterized in that a lead-silver alloys, especially Chromin, is used as the electrode material.

12. A process according to any one of claims 1-11, characterized in that the reaction is carried out in an undivided electrolysis cell, i.e. in a cell without a diaphragm.

13. A process according to any one of claims 1-12, characterized in that it is carried out in filter press cells, through cells or channel cells, especially in channel cells.

14. A process according to any one of claims 1-13, characterized in that the cell consists of an inert material, especially of synthetic materials based on polymers.

15. A process according to any one of claims 1-14, characterized in that the chemical oxidation is carried out in a three-phase system.

16. A process according to claim 15, characterized in that two of the three phases correspond to the system $Mn^{3+}(Mn^{2+})/H_2SO_4$ of the electrochemical oxidation.

17. A process according to claim 15 or 16, characterized in that the three-phase system consists of a suspension of solid $Mn^{2+}$ salt, or $Mn^{2+}$ salt and $Mn^{3+}$ salt in 40 to 90% $H_2SO_4$, especially in 50 to 65% $H_2SO_4$, and organic material.

18. A process according to claim 17, characterized in that the total amount of manganese salts in the inorganic phase amounts to $\geq$ 2.5 mol/l, especially 3 to 4 mol/l.

19. A process according to any one of claims 15-18, characterised in that p-tert.butyltoluene is used as the solvent.

20. A process according to claim 19, characterized in that the p-tert.butyltoluene is oxidized at a temperature of 90 to 100°C.

21. A process according to any one of claims 1-20, characterized in that it is carried out under a protective gas atmosphere, e.g. under $N_2$.

22. A process according to any one of claims 1-21, characterized in that the oxidation is discontinued after a conversion of $\leq$ 40%, especially 20 to 30%, of p-tert.butyltoluene.

23. A process according to any one of claims 1-22, characterized in that the separation of the reaction product consisting essentially of TBB, $MnSO_4$, $H_2SO_4$ and organic solvent into the inorganic phases and the organic phase is carried out by filtration and decantation or by centrifugation.

24. A process according to any one of claims 1-23, characterized in that the inorganic phases are freed from organic material, especially by stripping by means of steam or an inert gas, before the recycling into the electrolysis cycle.

25. A process according to any one of claims 1-24, characterized in that the purified inorganic phases are recycled into the electrolysis cycle in place of fresh electrolyte.

26. A process according to any one of claims 1-25, characterized in that the electrochemical oxidation is carried out continuously and the chemical oxidation is carried out continuously or discontinuously.

**Revendications**

1. Procédé de préparation du p-tert.-butylbenzaldéhyde, caractérisé en ce que l'on oxyde le p-tert.-butyltoluène à l'aide d'un sel de $Mn^{+3}$ à des températures de 80 à 110°C, ce sel ayant été obtenu par oxydation électrochimique d'un sel de $Mn^{+2}$ à des températures de 60 à 110°C et à des densités de courant supérieures ou égales à 100 $mA/cm^2$, l'oxydation chimique et l'oxydation électrochimique étant effectuées dans des récipients de réaction séparés.

2. Procédé selon la revendication 1, caractérisé en ce que l'on opère par utilisation du sulphate de manganèse.

3. Procédé selon la revendication 1 ou 2, caractérisé en ce que l'oxydation électrochimique est effectuée dans de l'acide sulfurique à une concentration de 40 à 90%, plus spécialement de 50 à 65%.

4. Procédé selon l'une des revendications 1 à 3, caractérisé en ce que l'on soumet le $Mn^{+2}$ à oxydation électrochimique à une température de 80 à 100°C.

5. Procédé selon l'une quelconque des revendications 1 à 4, caractérisé en ce que le sel de $Mn^{+2}$ est présent à une concentration de 1 à 5 moles/litre, plus spécialement de 3,0 à 4,0 moles/litre dans la phase inorganique.

6. Procédé selon l'une des revendications 1 à 5, caractérisé en ce que l'on opère à de fortes densités de courant, de 300 à 500 $mA/cm^2$.

7. Procédé selon l'une des revendications 1 à 6, caractérisé en ce que l'on opère à une tension sur la cellule d'environ 2,5 à 4 V, plus spécialement de 2,5 à 3,0 V.

8. Procédé selon l'une des revendications 1 à 7, caractérisé en ce que l'on utilise en tant que matériau d'anode du graphite vitreux, du plomb, des alliages de plomb, des métaux nobles comme le platine ou des métaux revêtus de métal noble, passivés contre une corrosion anodique, mais plus spécialement des alliages de plomb.

9. Procédé selon l'une des revendications 1 à 8, caractérisé en ce que l'on utilise en tant que matériau de cathode du graphite vitreux, du plomb, des alliages de plomb ou des métaux nobles, mais plus spécialement du plomb ou des alliages de plomb.

10. Procédé selon la revendication 8 ou 9, caractérisé en ce que l'anode et la cathode consistent en le même matériau.

11. Procédé selon la revendication 10, caractérisé en ce que l'on utilise en tant que matériau d'électrode un alliage plomb/argent, en particulier la chromine.

12. Procédé selon l'une des revendications 1 à 11, caractérisé en ce que l'on effectue la réaction dans une cellule d'électrolyse non compartimentée, c'est-à-dire dans une cellule sans diaphragme.

13. Procédé selon l'une des revendications 1 à 12, caractérisé en ce que l'on opère dans des cellules à filtres-presses, des cellules à augets ou des cellules à canaux, en particulier dans des cellules à canaux.

14. Procédé selon l'une des revendications 1 à 13, caractérisé en ce que la cellule consiste en un matériau inerte, en particulier une résine synthétique à base de polymère.

15. Procédé selon l'une des revendications 1 à 14, caractérisé en ce que l'oxydation chimique est effectuée dans un système à trois phases.

16. Procédé selon la revendication 15, caractérisé en ce que deux des trois phases correspondent

au système $Mn^{+3}(Mn^{+2})/H_2SO_4$ de l'oxydation électrochimique.

17. Procédé selon la revendication 15 ou 16, caractérisé en ce que le système à trois phases consiste en une suspension de sel solide de $Mn^{+2}$ ou de $Mn^{+2}$ et de $Mn^{+3}$ dans de l'acide sulfurique à une concentration de 40 à 90%, plus spécialement de l'acide sulfurique à une concentration de 50 à 65%, et une matière organique.

18. Procédé selon la revendication 17, caractérisé en ce que la quantité totale de sels de manganèse dans la phase inorganique est supérieure ou égale à 2,5 moles/litre et plus spécialement égale à 3-4 moles/litre.

19. Procédé selon l'une des revendications 15 à 18, caractérisé en ce que l'on utilise le p-tert.butyltoluène en tant que solvant.

20. Procédé selon la revendication 19, caractérisé en ce que l'on oxyde le p-tert.-butyltoluène à une température de 90 à 100°C.

21. Procédé selon l'une des revendications 1 à 20, caractérisé en ce que l'on opère en atmosphère de gaz protecteur, par exemple d'azote.

22. Procédé selon l'une des revendications 1 à 21, caractérisé en ce que l'on interrompt l'oxydation après un taux de conversion du p-tert.-butyltoluène inférieur ou égale à 40%, et plus spécialement de 20 à 30%.

23. Procédé selon l'une des revendications 1 à 22, caractérisé en ce que le produit de réaction des phases inorganiques et de la phase organique, consistant essentiellement en TBB, $MnSO_4$, $H_2SO_4$ et solvant organique est séparé par filtration et décantation ou par centrifugation.

24. Procédé selon l'une des revendications 1 à 23, caractérisé en ce que les phases inorganiques sont débarrassées de la matière organique avant recyclage dans le circuit d'électrolyse, en particulier par entraînement à l'aide de vapeur d'eau ou d'un gaz inerte.

25. Procédé selon l'une des revendications 1 à 24, caractérisé en ce que les phases inorganiques purifiées sont recyclées dans le circuit d'électrolyse à la place de l'électrolyte frais.

26. Procédé selon l'une des revendications 1 à 25, caractérisé en ce que, à l'oxydation électrochimique, on opère en continu, et à l'oxydation chimique, on opère en continu ou en discontinu.